(19) Europäisches Patentamt
European Patent Office
Office européen des brevets

(11) Veröffentlichungsnummer: **0 147 526**
**B1**

(12) **EUROPÄISCHE PATENTSCHRIFT**

(45) Veröffentlichungstag der Patentschrift: **28.12.88**

(51) Int. Cl.⁴: **A 61 K 31/725**

(21) Anmeldenummer: **84109890.8**

(22) Anmeldetag: **20.08.84**

(54) **Verfahren zur Erhöhung der enteralen Resorbierbarkeit von Heparin sowie das so erhältliche Heparinpräparat.**

(30) Priorität: **27.08.83 DE 3331009**

(43) Veröffentlichungstag der Anmeldung:
**10.07.85 Patentblatt 85/28**

(45) Bekanntmachung des Hinweises auf die Patenterteilung:
**28.12.88 Patentblatt 88/52**

(84) Benannte Vertragsstaaten:
**AT BE CH DE FR GB IT LI NL SE**

(56) Entgegenhaltungen:
**BE-A- 860 011**
**DE-A- 2 745 943**
**DE-B- 1 492 011**
**FR-A- 2 381 520**
**GB-A- 1 381 426**
**US-A- 4 021 544**
**US-A- 4 021 545**
**US-A- 4 066 829**

(73) Patentinhaber: **BASF Aktiengesellschaft,**
**Carl-Bosch-Strasse 38, D-6700 Ludwigshafen (DE)**

(72) Erfinder: **Herr, Dieter, Dr., Hardenburgstrasse 19,**
**D-6701 Altrip (DE)**

**EP 0 147 526 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die Erfindung betrifft die Herstellung von einem Heparinpräparat mit erhöhter enteraler Resorbierbarkeit, das so erhältliche Heparinpräparat bzw. die Verwendung von bestimmten Tensiden zur Erhöhung der Resorbierbarkeit von Heparin.

Heparin ist eine lang bekannte Substanz. Als Heparin kommen auch niedermolekulare Heparine in Betracht, die nach bekannten Methoden, wie z.B. fraktionierte Fällung mit Lösungsmitteln oder Oxidation mit Perjodat hergestellt werden können. Spezielle Methoden, wie Gelfiltration (Carbohydr. Res. 51 (1976), 119, Carbohydr. Res. 21 (1972), 173), teilweise Depolymerisation durch Veresterung der Carboxyl-Gruppe und anschliessende β-Elimination (EPA 40 144), Affinitätschromatographie (Thrombosis Res. 9 (1976), 575, Spaltung mit Peroxiden (Adv. Carbohydr. Chem. 10 (1955), 359), Nitrit (Carbohydr. Res. 21 (1972), 173, Biochemistry 15 (1976), 3932) oder Heparinase (J. Biol. Chem. 248 (1973), 6408) sowie die Kombination der vorgenannten Methoden wurden ebenfalls zur Herstellung von niedermolekularem Heparin herangezogen.

Heparin wird seit etwa 40 Jahren zur Verhinderung von Thrombosen sowie zur Hemmung der Blutgerinnung eingesetzt. Der grosse Nachteil hierbei ist, dass Heparin nur i.v. oder s.c. appliziert werden kann. Dies beschränkt die Anwendung von Heparin weitgehend auf die Klinik und stellt eine nicht unerhebliche Belastung des klinischen Personals und der Patienten dar.

Es ist bereits versucht worden, Heparin mit Hilfe von Liposomen oral resorbierbar zu machen (BE-PS 860 011, FR-PS 2 492 259), doch konnten sich diese Verfahren wegen des erforderlichen hohen Aufwandes, der geringen Stabilität sowie der teilweise immunogenen Eigenschaften der Präparate nicht durchsetzen.

Es wurde nun gefunden, dass man Heparin durch den Zusatz von nichtionischen Tensiden enteral resorbierbar machen kann.

Gegenstand der Erfindung ist ein Verfahren zur Herstellung von einem Heparinpräparat in Form von Tabletten, Filmtabletten, Kapseln, Pulvern, Granulaten oder Dragees mit erhöhter enteraler Resorbierbarkeit, dadurch gekennzeichnet, dass man Heparin mit einem physiologisch verträglichen nichtionischen Tensid mischt, welches durch Umsetzung von Ethylenoxid mit einem Fettalkohol oder einem Alkylphenol gewonnen wird.

Gegenstand der Erfindung sind auch ein enteral resorbierbares Heparinpräparat in Form von Tabletten, Filmtabletten, Kapseln, Pulvern, Granulaten oder Dragees enthaltend Heparin und ein physiologisch verträgliches nichtionisches Tensid, welches durch Umsetzung von Ethylenoxid mit einem Fettalkohol oder einem Alkylphenol gewonnen wird und eine Verwendung von physiologisch verträglichen nichtionischen Tensiden, welche durch Umsetzung von Ethylenoxid mit einem Fettalkohol oder einem Alkylphenol gewonnen werden, als Zusatz in einem Heparinpräparat in Form von Tabletten, Filmtabletten, Kapseln, Pulvern, Granulaten oder Dragees, um dessen enterale Resorbierbarkeit zu erhöhen.

Als Heparin kann eines mit einem mittleren Molekulargewicht ($\bar{M}_w$) von etwa 1.200 bis zu etwa 30.000 verwendet werden. Je niedriger das Molekulargewicht des Heparins ist, desto stärker ist die resorptionsfördernde Wirkung der Zusatzstoffe. Als Heparin kommen auch relativ niedermolekulare Heparinfraktionen in Betracht (vgl. DE-OS 2 833 898), jedoch ist hierbei zu berücksichtigen, dass Heparinpräparationen mit einem Molekulargewicht von unter 3.000 schon selbst geringfügig resorbierbar sind; die Zugabe der Zusatzstoffe führt hier jedoch zu einer wesentlich verstärkten Resorption.

Das Heparin kann auch in Form seiner Salze mit physiologisch verträglichen Basen verwendet werden. Als Salze sind insbesondere die Na-, Ca- oder Mg-Salze zu verstehen. Auch Salze mit organischen Basen wie Diethylamin, Triethylamin oder Triethanolamin kommen in Betracht. Der Ausdruck Heparin – wie er hier verwendet wird – schliesst die Salze mit ein.

Als Tenside eignen sich solche, die nichtionisch (nichtionogen) und physiologisch verträglich sind und sich durch Umsetzen von Ethylenoxid mit Fettalkoholen oder Alkylphenolen gewinnen lassen. Hierzu gehören beispielsweise Verbindungen der Formel I:

$$R-[(CH_2)_2-O]_m-H \qquad I,$$

in der

R eine Alkyloxygruppe mit jeweils 4–22 Kohlenstoffatomen und

m eine ganze Zahl von 1–200

darstellen. Solche Produkte sind beispielsweise im Handel unter der Bezeichnung BRIJ®, CREMOPHOR® und TRITON® erhältlich.

Unter den Tensiden der Formel I sind insbesondere solche geeignet, in denen R einen gesättigten oder einen eine oder mehrere Doppelbindungen tragenden Kohlenwasserstoffrest mit 7–18, vorzugsweise 10–18 Kohlenstoffatomen darstellt, und m eine Zahl von 10–100 bedeutet. Beispiele hierfür sind insbesondere die Oxy-Reste, die durch Wasserstoffabspaltung aus Octanol (→Octyloxy), Nonanol, Decanol, Laurylalkohol, Cetylalkohol, Isohexadecylalkohol, Oleylalkohol oder Stearylalkohol entstehen.

Speziell seien neben den in den Beispielen erwähnten Resten für R die Oxy-Reste der Tenside Octylphenol und Nonylphenol genannt:

Verbindungen der Formel I lassen sich beispielsweise durch Umsetzen von Verbindungen der Formel ROH mit Ethylenoxid gewinnen.

Die enterale Resorption des Heparins beginnt bereits bei einem Zusatz von 0,02 Gewichtsteilen Tensid pro Gewichtsteil Heparin. Gute Ergebnisse werden nach Zugabe von 0,3–5 Gewichtsteilen Tensid pro Gewichtsteil Heparin erzielt. Ein höherer Tensidanteil führt zu keiner weiteren Verbesserung der Resorption des Heparins bzw. Heparinoids.

Die neuen Heparinpräparate werden nach oraler und rektaler Gabe gut resorbiert, sind einfach herstellbar und über einen langen Zeitraum stabil.

Die gute Resorption nach oraler Gabe zeigen folgende Versuche:

A. Jeweils 2 der gemäss den Beispielen 1a–1d erhaltenen Kapseln (Gesamtdosis 28.000 IU Heparin) wurden Beagle-Hunden (Gewicht etwa 7 kg) oral verabfolgt. Die Wirkstoffkonzentration im Blut der Hunde wurde vor sowie 30′, 1 h, 2 h, 3 h, 5 h, 8 h, 14 h und 24 h nach Heparingabe ermittelt. Die Heparinbestimmung erfolgte in Citratplasmen mit chromogenem Substrat (vgl. Thromb. Res. 8, 413 (1976) sowie Berichtsband 2. Kongress für Thrombose und Hömostase, Münster 1982, Schattauer-Verlag Stuttgart, S. 238–246). Ausserdem wurde die Thrombinzeit ermittelt (vgl. Kleiner Gerinnungstatus, Herausgeber Behringwerke 1982, S. 40). Nach 2 h war die Heparinkonzentration (c) im Blut und der Quotient T der Thrombinzeit der behandelten zu den unbehandelten Tieren am höchsten. Die Tabelle 1 zeigt die 2 Stunden nach Applikation erhaltenen Daten.

Tabelle 1

| Präparat des Beispiels | c | T |
|---|---|---|
| 1a | 0,3 IU/ml | 2 |
| 1b | 1,2 IU/ml | 5 |
| 1c | 2,5 IU/ml | 7 |
| 1d | 8,5 IU/ml | 15 |

Die in den Beispielen 1a und 1b verwendeten Heparine führten ohne Zusatz von Tensid nach oraler Gabe zu keinen messbaren Heparinkonzentrationen im Blut oder zu Veränderungen der Thrombinzeit. Dasselbe gilt für die Applikation der Tenside ohne Heparin.

Das in Beispiel 1c verwendete Heparin führt ohne Tensid-Zusatz zu einem Heparinspiegel im Blut von 0,15 IU/ml, jedoch zu keiner Veränderung der Thrombinzeit. Entsprechendes gilt für das in Beispiel 1d verwendete Heparin, jedoch lag hier der Heparinspiegel bei 0,25 IU/ml.

B. Analog A wurden Kapseln gemäss den Beispielen 2a–2i (Gesamtdosis 28.000 IU Heparin) Hunden appliziert und die relative resorptionsfördernde Wirkung im Vergleich zu Tensid-freien Präparaten gemessen.

Es zeigte sich, dass die Tenside die Resorption des Heparins um den Faktor 5 bis 100 erhöhen.

Entsprechende Daten wurden auch nach rektaler Gabe der Heparinpräparate erhalten. Hierzu wurden die Mischungen jedoch nicht in Kapseln gefüllt, sondern direkt den Hunden mit Hilfe einer Knopfsonde zugeführt.

Die neuen Zubereitungen sind somit in allen für das Heparin bekannten Indikationsgebieten, wie Thromboseprophylaxe, Antikoagulation, Lipidsenkung, Arteriosklerose, Entzündungshemmung und Tumorhemmung, anwendbar.

Das neue Heparinpräparat kann in üblicher Weise enteral verabfolgt werden. Die Dosierung hängt vom Alter, Zustand und Gewicht des Patienten ab. In der Regel beträgt die tägliche Wirkstoffdosis bezogen auf Heparin zwischen etwa 50 und 5000 IU/kg Körpergewicht. Im Normalfall werden mit täglichen Dosen von 100–2000 IU/kg zufriedenstellende Ergebnisse erzielt.

Das neue Heparinpräparat kann in den gebräuchlichen galenischen Applikationsformen fest oder flüssig angewendet werden, z. B. als Tabletten, Filmtabletten, Kapseln, Pulver, Granulate oder Dragees. Diese werden in üblicher Weise hergestellt. Die Wirkstoffe können dabei mit den üblichen galenischen Hilfsmitteln wie Tablettenbindern, Füllstoffen, Konservierungsmitteln, Tablettensprengmitteln, Fliessreguliermitteln, Weichmachern, Netzmitteln, Dispergiermitteln, Emulgatoren, Lösungsmitteln, Retardierungsmitteln und/oder Antioxidantien verarbeitet werden (vgl. H. Sucker et al: Pharmazeutische Technologie, Thieme-Verlag, Stuttgart, 1978).

Beispiel 1

a) 0,87 g handelsübliches Heparin ($\bar{M}_w$ = 13.000 Dalton) entsprechend 140.000 IU wurden mit 1,3 ml Wasser und 0,5 g Polyethylenglykol 400 als Lösungsvermittler vermengt und danach mit 1,20 g Polyoxyethylen-(20)-cetylether (Tensid) homogen gemischt und zu gleichen Gewichtsteilen in 10 Hartgelatine-Kapseln gebracht.

Entsprechend wurden je 10 Kapseln mit Heparin (14.000 IU pro Kapsel) vom Molekulargewicht b) $\bar{M}_w$ = 9000, c) $\bar{M}_w$ = 6.000 und d) $\bar{M}_w$ = 3.000 hergestellt.

Beispiel 2

Analog Beispiel 1d wurden Kapseln unter Verwendung folgender Tenside anstelle von Polyethylenglykol-(20)-cetylalkohol hergestellt.

a) Polyoxyethylen-(23)-laurylether
b) Polyoxyethylen-(2)-cetylether
c) Polyoxyethylen-(16)-cetylether
d) Polyoxyethylen-(20)-cetylether
e) Polyoxyethylen-(20)-stearylether
f) Polyoxyethylen-(20)-oleylether
g) Polyoxypropylen-(15)-stearylether
h) Octylphenolpolyoxyethylen-(16)-ether
i) Octylphenolpolyoxyethylen-(30)-ether

**Patentansprüche**

1. Verfahren zur Herstellung von einem Heparinpräparat in Form von Tabletten, Filmtabletten, Kapseln, Pulvern, Granulaten oder Dragees mit erhöhter enteraler Resorbierbarkeit, dadurch gekennzeichnet, dass man Heparin mit einem physiologisch verträglichen nichtionischen Tensid mischt, welches durch Umsetzung von Ethylenoxid mit einem Fettalkohol oder einem Alkylphenol gewonnen wird.

2. Enteral resorbierbares Heparinpräparat in Form von Tabletten, Filmtabletten, Kapseln, Pulvern, Granulaten oder Dragees, enthaltend Heparin und ein physiologisch verträgliches nichtionisches Tensid, welches durch Umsetzung von Ethylenoxid mit einem Fettalkohol oder einem Alkylphenol gewonnen wird.

3. Verwendung von physiologisch verträglichen nichtionischen Tensiden, welche durch Umsetzung von Ethylenoxid mit einem Fettalkohol oder einem Alkylphenol gewonnen werden, als Zusatz in einem Heparinpräparat in Form von Tabletten, Filmtabletten, Kapseln, Pulvern, Granulaten oder Dragees, um dessen enterale Resorbierbarkeit zu erhöhen.

**Claims**

1. A process for preparing a heparin product of increased enteral absorbability in the form of tablets, film tablets, capsules, powders, granules or coated tablets, which comprises mixing heparin with a physiologically tolerated nonionic surfactant obtained by reacting ethylene oxide with a fatty alcohol or an alkylphenol.

2. An enterally absorbable heparin product in the form of tablets, film tablets, capsules, powders, granules or coated tablets containing heparin and a physiologically tolerated nonionic surfactant obtained by reacting ethylene epoxide with a fatty alcohol or an alkylphenol.

3. Use of a physiologically tolerated nonionic surfactant obtained by reacting ethylene oxide with a fatty alcohol or alkylphenol as an additive in a heparin product in the form of tablets, film tablets, capsules, powders, granules or coated tablets to increase the enteral absorbability of the heparin.

**Revendications**

1. Procédé de préparation d'héparine, sous forme de comprimés, comprimés sur film, capsules, poudres, granulés ou dragées, à capacité de résorption intestinale élevée, caractérisé par le fait que l'on mélange de l'héparine avec un tenside non ionique, acceptable physiologiquement, qui est obtenu par réaction d'éthylène-oxyde avec un alcool gras ou un alkylphénol.

2. Préparation d'héparine résorbable par voie intestinale sous forme de comprimés, comprimés sur film, capsules, poudres, granulés ou dragées, contenant de l'héparine et un tenside non ionique, acceptable physiologiquement, qui est obtenu par réaction d'éthylène-oxyde avec un alcool gras ou un alkylphénol.

3. Utilisation de tensides non ioniques, acceptables physiologiquement, qui sont obtenus par réaction d'éthylène-oxyde avec un alcool gras ou un alkylphénol, comme additif dans une préparation d'héparine, sous forme de comprimés, comprimés sur film, capsules, poudres, granulés ou dragées, pour élever la capacité de résorption intestinale de cette préparation.